# EUROPEAN PATENT APPLICATION

(11) **EP 4 321 174 A1**
(43) Date of publication of application: **14.02.2024**
(21) Application number: 22785005.4
(22) Date of filing: 07.04.2022
(51) Int. Cl.: A61K 39/395, A61K 48/00, A61K 31/713, A61P 35/00, A61P 37/02, G01N 33/50, C07K 16/28, C07K 14/78, A61K 39/00

(54) **NOVEL TARGET FOR ANTI-CANCER EFFECT AND IMMUNITY ENHANCEMENT**

(30) Priority: 09.04.2021 KR 20210046810
(71) Applicant: Genome and Company, Seongnam-si, Gyeonggi-do 13486 (KR)
(72) Inventor: JEON, Bu-Nam, Seongnam-si Gyeonggi-do 13486 (KR); CHA, Mi Young, Seongnam-si Gyeonggi-do 13486 (KR)
(74) Representative: Rückerl, Florian
(86) International application number: PCT/KR2022/005073
(87) International publication number: WO 2022/216096

(57) **Abstract**

The present disclosure relates to a pharmaceutical composition for treating or preventing cancer, a pharmaceutical composition for immune-enhancing, a method of screening of anti-cancer agent, and a method of providing information necessary for analysis of cancer prognosis. According to the present disclosure, cancer can be effectively prevented or treated using a CNTN4 inhibitor, and the immunity of subject can be enhanced. In addition, the present disclosure is very useful because it can effectively select new anticancer drug candidates by determining the activity or expression of CNTN4 and can provide information necessary for diagnosing and predicting cancer prognosis.

## Description

### [Technical Field]

The present disclosure provides a pharmaceutical composition for treating or preventing cancer, comprising an inhibitor of CNTN4. In addition, the present disclosure provides a pharmaceutical composition for immune-enhancing, comprising an inhibitor of CNTN4. Furthermore, the present disclosure provides a method of screening of anti-cancer agent using CNTN4, and a method of providing information necessary for analysis of cancer prognosis using CNTN4.

### [Background Art]

Despite advances in understanding the etiology of cancer and the methods for treating cancer over the past several years, it is still the leading cause of death worldwide. Although anti-cancer treatments exist for many malignancies, such treatments often do not fully control such malignancies or are not effective in all patients. Most of the methods currently being used to treat cancer are relatively non-selective. The affected tissue is removed through surgery, the size of solid tumors is reduced through radiation therapy, or chemotherapy is used to kill cancer cells rapidly. In particular, the chemotherapy can cause the drug resistance, and sometimes restricts the administrable dose. It causes severe side effects so that they may rule out the use of potentially effective agents. Accordingly, there is a need to develop more target-specific and effective cancer therapies.

The adaptive immune system of the human is a very precise system which is able to specifically remove cancer cells. In particular, T cells determine cell mediated adaptive immunity, and recognize and remove non-self antigens or abnormal antigens that a cell is exposed to. T cells express about 20,000 to 40,000 TCR molecules per cell, and recognize several antigens (determined by their peptide sequences) among the 100,000 pMHC molecules of APC to begin signal transfer. Such TCR molecules should function as highly sensitive sensors which need to recognize very minute changes in the antigen and transfer signals. This cell-mediated adaptive immunity operates in a very precise manner to effectively remove cancer cells. If an antigen-specific adaptive immune system does not operate normally, serious problems are caused in the ability to remove cancer cells. For example, if the protein PD-L1 or PD-L2 on the surface of a cancer cell binds to the protein PD-1 on the surface of a T cell, the T cell is not able to attack cancer cells. Therefore, for effective cancer treatment, it is necessary to remove the factors that hinder T cell's ability to remove cancer cells.

Accordingly, the inventors have conducted research to develop a method of cancer treatment using the human immune system, and identified that inhibition of the activity and expression of CNTN4 leads to substantial suppression of development, growth, invasion and metastasis of cancer.

### [Technical Solution]

To achieve the purposes of the present disclosure, one aspect of the present disclosure provides a pharmaceutical composition for treating or preventing cancer comprising an inhibitor of CNTN4 as an active ingredient.

The term "CNTN4 (Contactin-4)" refers to a protein that is encoded by *CNTN4* gene, which belongs to the immunoglobulin superfamily. It is reported to be a glycosylphosphatidylinositol (GPI)-anchored neuronal membrane protein that functions as a cell adhesion molecule, and to be involved in formation of axon connections in developing nervous system.

The CNTN4 may be human-derived CNTN4. More specifically, the amino acid sequence of CNTN4 may comprise the sequence of NCBI Reference Sequence: NP_783200.1 disclosed in NCBI. In addition, the amino acid sequence of CNTN4 may be or comprise, but is not limited to, amino acid sequences having at least 80%, 85%, 90% or 95% identity with each sequence of NCBI Reference Sequence: NP_783200.1, as well as amino acid sequences having the property or function of CNTN4.

The gene of CNTN4 may be or comprise a nucleic acid sequence encoding the amino acid sequence of human-derived CNTN4, or the nucleic acid sequence of NCBI Reference Sequence: NM_175607.3 disclosed in NCBI. In addition, the nucleic acid sequence of CNTN4 may be or comprise, but are not limited to, nucleic acid sequences having at least 80%, 85%, 90% or 95% identity with each sequence of NCBI Reference Sequence: NM_175607.3, as well as nucleic acid sequences that can produce amino acids having the property or function of CNTN4.

The term "an inhibitor of CNTN4" refers to substances that inhibit the activity or expression of CNTN4.

The inhibitor of CNTN4 can preferably suppress the function of cancer cells evading T cells. The inhibitor of CNTN4 block the activity of CNTN4 existing in a cancer cell, thereby suppressing the mechanism that T cells are rendered unable to attack cancer cells by CNTN4 and maintaining the immune activity of T cells against cancer cells. Alternatively, the inhibitor of CNTN4 specifically bind to CNTN4 protein, and interfere with binding of CNTN4 to T cells. Alternatively, the inhibitor of CNTN4 suppress a particular metabolic pathway of CNTN4 to reduce the expression of protein, or cause CNTN4 to denature so that the protein loses its activity. Therefore, the inhibitor of CNTN4 according to the present disclosure are very effective in treating or preventing cancer.

The inhibitor of CNTN4 may include, but are not limited to, any compounds, proteins, fusion proteins, antibodies, amino acids, peptides, viruses, carbohydrates, lipids, nucleic acids, extracts or fractions so long as it inhibits the activity or expression of CNTN4.

In one embodiment, the inhibitor of CNTN4 are ones that reduce the expression of CNTN4 in a cancer cell compared to a cancer cell not treated with inhibitor of CNTN4. Reduction in expression of CNTN4 may refer to lowered or no level of mRNA and/or protein produced from CNTN4 gene. The inhibitor of CNTN4 may include, but are not limited to, antisense nucleic acid, siRNA, shRNA, miRNA, ribozyme, etc. which binds in a complementary manner to DNA or mRNA of CNTN4 gene.

The term "antisense nucleic acid" refers to DNAs or RNAs comprising nucleic acid sequences complementary to the sequence of certain mRNA, or fragments or derivatives thereof, which bind to or hybridize with the complementary sequences in mRNA and inhibit the translation of mRNA into protein.

The term "siRNA (small interfering RNA)" refers to a short double chain RNA which is able to induce the RNAi (RNA interference) through cleavage of certain mRNA. The siRNA comprises a sense RNA strand having a sequence homologous to the mRNA of the target gene, and an antisense RNA strand having a sequence complementary thereto. The siRNA can inhibit the expression of the target gene, and thus can be used in gene knockdown, genetic therapy, etc.

The term "shRNA (short hairpin RNA)" is a single strand RNA, which comprises a stem portion forming a double strand portion through hydrogen bonds, and a loop portion. It is processed by a protein such as Dicer to be converted into siRNA, and performs the same function as siRNA.

The term "miRNA (micro RNA)" refers to 21 to 23 non-coding RNAs which modulate gene expression after transcription by promoting the degradation of target RNA or by suppressing its translation.

The term "ribozyme" refers to an RNA molecule that has an enzyme-like function, recognizing a particular base sequence and cutting the same. The ribozyme comprises an area that specifically binds to a complementary base sequence of a target messenger RNA strand, and an area that cleaves the target RNA.

The antisense nucleic acid, siRNA, shRNA, miRNA, ribozyme, etc. that binds complementarily to the DNA or mRNA of CNTN4 gene can inhibit the translation of mRNA of CNTN4, its translocation into the cytoplasm, its maturation, or any other activities crucial for the biological functions of CNTN4.

In one embodiment, the inhibitor of CNTN4 are ones that deactivate the function of CNTN4 or reduce the activity thereof in a cancer cell compared to a cancer cell not treated with inhibitor of CNTN4. The inhibitor of CNTN4 may include, but are not limited to, compounds, peptides, peptide mimetics, fusion proteins, antibodies, aptamers, etc. that bind specifically to CNTN4 protein.

The term "specific" or "specifically" refers to the ability to bind to only a target protein without affecting other proteins in the cell.

The term "antibody" may include monoclonal antibodies, polyclonal antibodies, bispecific antibodies, multispecific antibodies, chimera antibodies, humanized antibodies and human antibodies, and may also include new antibodies as well as antibodies known to the art or commercialized in the art. The antibody may include not only the forms having a full length comprising two heavy chains and two light chains but also the functional fragments of antibody molecules, so long as they specifically bind to CNTN4. The functional fragment of antibody molecule refers to a fragment having at least its antigen-binding function, and may include, but are not limited to, Fab, F(ab'), F(ab')2, Fv, etc.

The term "peptide mimetics" refers to a peptide or non-peptide which inhibits the binding domain of protein of CNTN4 that induces CNTN4 activities.

The term "aptamer" refers to a single strand nucleic acid (DNA, RNA or modified nucleic acid) having in itself a stable tertiary structure and being able to bind to a target molecule with high affinity and specificity.

The substance inhibiting the activity or expression of CNTN4 which is comprised in the pharmaceutical composition of the present disclosure can inhibit suppression of T cell function by CNTN4, and accordingly can increase or maintain the ability of T cells to attack and kill cancer cells. Here, the ability of T cells to attack and kill cancer cells in a group treated with inhibitor of CNTN4 may be increased by 5% to 200% as compared to a group not treated with inhibitor of CNTN4. Thus, the pharmaceutical composition of the present disclosure can be useful in the preventing or treating cancer.

The cancer that can be treated or prevented by the pharmaceutical composition of the present disclosure may include, but are not limited to, stomach cancer, lung cancer, liver cancer, colorectal cancer, colon cancer, small intestinal cancer, pancreatic cancer, brain cancer, bone cancer, melanoma, breast cancer, sclerosing adenosis, uterine cancer, cervical cancer, head and neck cancer, esophageal cancer, thyroid cancer, parathyroid cancer, renal cancer, sarcoma, prostate cancer, urethral cancer, bladder cancer, blood cancer, leukemia, lymphoma, fibroadenoma, etc.

The pharmaceutical composition according to the present disclosure may comprise the active ingredient alone, or may additionally comprise one or more pharmaceutically acceptable carriers, excipients, diluents, stabilizing agents, preserving agent, etc.

The Pharmaceutically acceptable carriers may include, for example, carriers for oral administration or non-oral administration. The carriers for oral administration may include, for example, lactose, starch, cellulose derivatives, magnesium stearate, stearic acid, etc. The carriers for non-oral administration may include, for example, water, suitable oils, saline, aqueous glucose, glycols, etc. The pharmaceutically acceptable stabilizing agents may include, for example, antioxidants such as sodium bisulfate, sodium sulfite or ascorbic acid. The pharmaceutically acceptable preserving agents may include, for example, benzalkonium chloride, methyl- or propyl-paraben, chlorobutanol, etc. Other pharmaceutically acceptable carriers may be those disclosed in the literature "Remington's Pharmaceutical Sciences, 19th ed., Mack Publishing Company, Easton, PA, 1995".

The pharmaceutical composition of the present disclosure may be administered to animals including human using various methods. For example, it may be administered orally or parenterally. The parenteral administration may include, but are not limited to, intravenous, intramuscular, intraarterial, intramarrow, intradural, percutaneous, subcutaneous, intraperitoneal, intranasal, intraintestinal, topical, sublingual, rectal administration, etc.

The pharmaceutical composition of the present disclosure may be prepared into formulations for oral or parenteral administration, depending on the administration route as described in the above.

The formulation for oral administration may be prepared in the form of powders, granules, tablets, pills, sugar-coated pills, capsules, liquids, gels, syrups, slurries, suspensions, etc., using methods known in the art. For example, the active ingredient of the present disclosure may be mixed with suitable excipient(s) and/or adjuvant(s), and then processed into a granule mixture to obtain a tablet or a sugar-coated tablet for oral administration. Examples of suitable excipients may include, but are not limited to, sugars including lactose, dextrose, sucrose, sorbitol, mannitol, xylitol, erythritol, maltitol, etc., starches including corn starch, wheat starch, rice starch, potato starch, etc., celluloses including cellulose, methyl cellulose, sodium carboxymethylcellulose, hydroxypropylmethylcellulose, etc., and fillers such as gelatin, polyvinyl pyrrolidone, etc. Optionally, disintegrating agents such as crosslinked polyvinyl pyrrolidone, agar, alginic acid or sodium alginate may be added. Further, the pharmaceutical composition of the present disclosure may further comprise anticoagulants, lubricants, wetting agents, aromatic agents, emulsifiers and preservatives, etc.

The formulation for parenteral administration may be prepared in the form of injections, gels, aerosols, nasal inhalers using methods known in the art.

These administration forms may refer to those disclosed in the literature known in the art "Remington's Pharmaceutical Science, 15th Edition, 1975. Mack Publishing Company, Easton, Pennsylvania 18042, Chapter 87: Blaug, Seymour".

The total effective dose of the pharmaceutical composition according to the present disclosure may be administered to a subject in a single dose, or in multiple doses through a fractionated treatment protocol.

The appropriate dose of the pharmaceutical composition according to the present disclosure or the contents of active ingredient in the pharmaceutical composition may be determined considering various factors such as administration route, times administered, patient age, body weight, health, gender, severity of disease, diet and excretion rate, etc. by a person having ordinary skill in the art. For example, the total dose of the pharmaceutical composition according to the present disclosure may be about 0.01µg to 1,000mg per 1kg body weight of a patient per day, or 0.1µg to 100mg. There is no particular limit to the dosage form, administration route and administration method, so long as the pharmaceutical composition shows the effect of the invention.

Another aspect of the present disclosure provides a pharmaceutical composition for immune-enhancing in a subject, comprising an inhibitor of CNTN4 as an active ingredient.

When the pharmaceutical composition is administered to a subject in need thereof, it can fully or partially reduce the expression or activity of CNTN4 in the subject to increase the level of T cell-mediated immune response.

Accordingly, the pharmaceutical composition of the present disclosure can be used for immune-enhancing. For example, it can be used for the subject in need of prevention, treatment or improvement of diseases related to immunodeficiency, decreased immunity, or immune system damage.

Another aspect of the present disclosure is to provide a use of an inhibitor of CNTN4 for treating or preventing cancer. And also, another aspect of the present disclosure is to provide a method of treating or preventing cancer in a subject, comprising administering to the subject an inhibitor of CNTN4. And also, another aspect of the present disclosure provides a method of immune-enhancing in a subject, comprising administering to the subject an inhibitor of CNTN4. In these methods, unless specifically mentioned otherwise, the terms associated have the same meaning as the terms explained for the pharmaceutical compositions in the above.

Another aspect of the present disclosure is to provide a method of screening an anticancer agent comprising:
(a) treating a cancer cell with a candidate of anti-cancer agent; and
(b) measuring the expression or activity of one or more of CNTN4 in the cancer cell.

Optionally, the method of screening an anti-cancer agent may further comprise a step of determining the candidate anti-cancer agent to be the anti-cancer agent if a group treated with the candidate anti-cancer agent shows a lower level of expression of CNTN4 mRNA or protein or a lower level of suppression of T cell activity by CNTN4 compared to a group not treated with the candidate anti-cancer agent. Here, the lower level may mean the significantly lower level. For example, the lower or significantly lower level may indicate an amount decreased by 5% to 95% (e.g., 10%, 15%, 20%, 25%, 30%, 35%, 40%, 45%, 50%, 55%, 60%, 65%, 70%, 75%, 80%, 85% and 90%). The group not treated with the candidate anti-cancer agent may be cancer cells to which no substance is added, or to which any substance such as anti-cancer agent other than an inhibitor of CNTN4 is treated.

The term "screening" refers to finding the target materials having the particular properties such as sensitivity or activity among proteins, fusion proteins, antibodies, peptides, antibiotics, enzymes, compounds or any other substances.

The term "candidate anti-cancer agent" may refer to a nucleic acid, protein, antibody, compound, extract or natural substance that is randomly selected or is thought to be able to inhibit the expression or activity of CNTN4 according to the usual selection method. The candidate of anti-cancer agent may preferably be a substance that inhibits the expression and/or activity of CNTN4.

The expression or activity of CNTN4 may be measured by determining the level of expression of the mRNA or protein of CNTN4, or by determining the degree to which T cell activity is suppressed by CNTN4.

The method of determining the level of expression of the mRNA of CNTN4 may include, but are not limited to, any method conventionally known to the art such as reverse transcriptase PCR, competitive reverse transcriptase PCR, real-time reverse transcriptase PCR, RNase protection assay, Northern blotting, DNA chip or RNA chip.

The method of determining the level of expression of CNTN4 protein may include, but are not limited to, any method conventionally known to the art such as Western blot, ELISA, radioimmunoassay analysis, radial immunodiffusion, Ouchterlony immunodiffusion, rocket immunoelectrophoresis, tissue immunohistochemistry, immunoprecipitation assay, complement fixation assay, FACS or protein chip.

The method of determining the degree of T cell activity inhibition by CNTN4 may include, but are not limited to, any method conventionally known to the art such as RT-PCR, Western Blot, ELISA, radioimmunoassay, radioimmunodiffusion, Ouchterlony immunodiffusion, rocket immunoelectrophoresis, immunohistochemistry, immunoprecipitation, complete fixation assay, or FACS.

In addition, in the method of screening of the present disclosure, confirmation of CNTN4 activity inhibition may be performed using conventional methods such as reacting CNTN4 protein and a candidate substance to measure an activity, yeast two-hybrid, searching for phage-displayed peptide clones binding to CNTN4 protein, HTS (high throughput screening) using natural material and chemical libraries, drug hit HTS, cell-based screening, or DNA array-based screening.

The method of screening an anti-cancer agent may be performed either in vitro or in vivo. For in vivo, the step of treating a cancer cell with a candidate of anti-cancer agent may be substituted by a step of administering a candidate of anti-cancer agent to a subject having cancer cells or suffering from cancer. Such a subject may be an animal such as human, mouse, etc.

The method of screening an anti-cancer agent is based on the novel disclosure in the present invention that inhibition of the activity or expression of CNTN4 can suppress the function of cancer cells evading T cells. The method of screening of the present disclosure is very advantageous in that it allows for easy development of new anti-cancer agents through a simple and inexpensive method.

Another aspect of the present disclosure provides a method of providing information necessary for analysis of cancer prognosis, comprising measuring expression or activity of CNTN4 in cells or tissues isolated from a subject.

In the method, the terms associated with the expression or activity of CNTN4 and its measurement, unless specifically mentioned otherwise, have the same meaning as the terms explained for the composition and the screening method.

The term "prognosis" refers to predictions as to progress of disease, improvement of disease, recurrence of disease, metastasis, and likelihood of death. For example, in the present disclosure, the prognosis refers to the possibility of curing a cancer patient or improving the condition of cancer patient.

The cell or tissue isolated from the subject may be a cancer cell or a tissue wherein cancer have occurred or cancer cells exist.

The method of providing information necessary for analysis of cancer prognosis is based on the fact that the lower activity or expression of CNTN4 in cancer cells can increase T cell activity and proliferation, thereby increasing cancer treatment effect.

Another aspect of the present disclosure provides a pharmaceutical composition for treating or preventing cancer comprising an anti-CNTN4 antibody or antigen-binding fragment thereof that specifically binds to CNTN4. More specifically, the pharmaceutical composition may comprise an anti-CNTN4 antibody or antigen-binding fragment thereof that binds to the Fibronectin 2 domain of CNTN4 when binding to CNTN4. And also, the pharmaceutical composition may be used for anti-cancer purposes.

The anti-CNTN4 antibody or antigen-binding fragment thereof binds to Fibronectin 2 (FN2) domain of CNTN4 when binding to CNTN4. The Fibronectin 2 domain may comprise an amino acid sequence of SEQ ID NO: 13.

CNTN4 consists of ten domains (such as Ig1 to Ig6, and FN1 to FN4) and binds to amyloid precursor protein (APP) in the body (Ig; Ig like domain, FN; fibronectin). More specifically, FN1 to FN2 domains (domain 7 to domain 8) of CNTN4 are strongly bound to KPI (Kunitz-type protease inhibitor) domain region of APP. FN1 to FN2 domains (domain 7 to domain 8) of CNTN4 are composed of an amino acid sequence of SEQ ID NO: 9, and KPI domain of APP is composed of an amino acid sequence of SEQ ID NO: 10.

When the anti-CNTN4 antibody or antigen-binding fragment thereof binds to CNTN4, binding of CNTN4 to APP is blocked or inhibited. Accordingly, the anti-CNTN4 antibody or antigen-binding fragment thereof may be used for inhibiting an interaction between CNTN4 and APP.

The articles "a" and "an" are used herein to refer to one or to more than one (i.e. to at least one) of the grammatical object of the article. By way of example, "an ingredient" means one ingredient or more than one ingredient. The term "A, B and/or C" is used herein to refer to A, or B, or C, or A and B, or A and C, or B and C, or A, B and C.

### [Brief Description of Figures]

FIG. 1 shows the proliferation (%) of CD4+ T cells suppressed by CNTN4.
FIG. 2 shows the proliferation (%) of CD8+ T cells suppressed by CNTN4.
FIGS. 3a to 3d show the cytotoxicity (%) of PBMC when lung cancer cell line A549 and PBMC were treated with CNTN4 inhibitors.
FIGS. 4a to 4d show the cytotoxicity (%) of PBMC when colon cancer cell line HCT-116 and PBMC were treated with CNTN4 inhibitors.
FIGS. 5a to 5d show the cytotoxicity (%) of PBMC when breast cancer cell line MDA-MB-231 and PBMC were treated with CNTN4 inhibitors.
FIGS. 6a to 6d show the cytotoxicity (%) of PBMC when gastric cancer cell line MKN-74 and PBMC were treated with CNTN4 inhibitors.
FIGS. 7a to 7d show the cytotoxicity (%) of PBMC when leukemia cell line U937 and PBMC were treated with CNTN4 inhibitors.
FIG. 8 shows the change of tumor size in mouse treated with CNTN4 inhibitors.
FIG. 9 shows the structures of CNTN4 and APP produced according to Example 4. (a) Four deletion structures of CNTN4 (#10: full length). (b) Structures of the three isoforms of APP (APP770: full length).
FIG. 10 shows the images obtained by SDS-PAGE and Western blotting according to Example 4.
FIG. 11 shows the amino acid sequences of the FN1-FN2 domain of CNTN4 and the KPI domain of APP.
FIG. 12 shows the amino acid sequences of the light chain variable region and heavy chain variable region of anti-CNTN4 antibody AB 1.
FIG. 13 is a schematic diagram of the Human CNTN4 domain sample prepared according to Example 5.2.1.
FIG. 14 shows the results of showing the binding ability between anti-CNTN4 antibody AB1 and CNTN4 domain sample according to Example 5.2.2.
FIG. 15 shows the amino acid sequence of Domain 8 (FN2 domain) of CNTN4.
FIG. 16 shows that anti-CNTN4 antibody AB1 binds to CNTN4 competitively with APP.

### [EXAMPLES]

In the following, exemplary embodiments of the inventive concept will be explained in further detail with reference to examples. However, the following examples are meant to exemplify the present invention, and the scope of the invention is not restricted by these examples.

### Example 1. Inhibition of the proliferation and activity of T cells

This example is to confirm whether CNTN4 suppress the proliferation and activity of the T cell, and ensures that cancer cells evade the T cell-mediated immune system.

### 1.1. Preparation of CD4+ cells and CD8+ T cells

Human blood was placed in a 10 ml tube coated with EDTA (or heparin) and mixed with PBS at a ratio of 1:1. Ficoll-Paque PLUS was placed in a 50 ml tube, and then the blood sample was added. After centrifugation, human PBMCs (peripheral blood mononuclear cells) were collected. The resultant was centrifuged, and the supernatant was removed. Then, RBC lysis (1x) was added, pipetted, and stored on ice for 3 minutes. After that, 50 ml of 10% FBS RPMI1640 was added, and the mixture was centrifuged to remove the supernatant. Then, FACS buffer was added, and the supernatant was removed by centrifugation. Subsequently, 50 ml of MACS buffer (PBS containing 0.5% bovine serum albumin and 2mM EDTA) was added, the number of cells was counted, and the supernatant was completely removed after centrifugation.

CD4+ T cells and CD8+ T cells were resuspended using 40 µl of MACS buffer based on the number of 1×10⁷ cells in a 50 ml tube. 10 µl of anti-CD4 and anti-CD8 biotin antibodies were added to the tube respectively, and then stored in the refrigerator for 5 minutes. Subsequently, 30 µl of MACS buffer based on the number of 1×10⁷ cells was added to the resultant, and 20 µl of anti-biotin microbeads were added and mixed. Then, CD4+ T cells and CD8+ T cells were separated using LS column, and were counted.

The prepared CD4+ T cells and CD8+ T cells were mixed with 1 µl of CFSE (carboxyfluorescein succinimidyl ester) based on the number of 2×10⁶ cells, and stored at 37°C for 3 minutes. Then, FBS was added into tubes containing the CD4+ T cells and CD8+ T cells respectively, and stored on ice 10 minutes. Thereafter, the supernatant was removed by centrifugation. The resultant was added with 30 ml of FACS buffer, pipetted, and centrifuged to remove the supernatant. Then, the resultant was mixed with 10 ml of 10% FBS RPMI1640, and the number of cells was counted.

### 1.2. Inhibition of T-cell activity by CNTN4

The recombinant human IgG1 Fc protein (Cat. No. 110-HG) and the recombinant human PD-L1/B7-H1 Fc chimera protein (Cat. No. 156-B7) were purchased from R&D systems. The recombinant human CNTN4 His Tag protein (Cat. No. 2205-CN) was purchased from R&D systems.

7.5 µg/ml or 10 µg/ml of each protein was mixed with 2.5 µg/ml of anti-CD3 antibody (BioLegend, Cat. No. 317325) in PBS, respectively. The resultant mixture was coated on 96-well plates at 4°C, and the wells were washed three times with PBS.

The CD4+ T cells and CD8+ T cells prepared in the Example 1.1 were added to each well of the 96-well plate at the number of 2×10⁶ cells in an amount of 200 µl, and then incubated.

CD4+ T cells and CD8+ T cells were activated by anti-CD3 antibody for 72 hours. The proliferation of CD4+ T cells and CD8+ T cells can be confirmed by the degree of CFSE fluorescent cell staining, and was analyzed by flow cytometry using FACSDiVa software (BD Biosciences).

### 1.3. Inhibition of T-cell activity by CNTN4

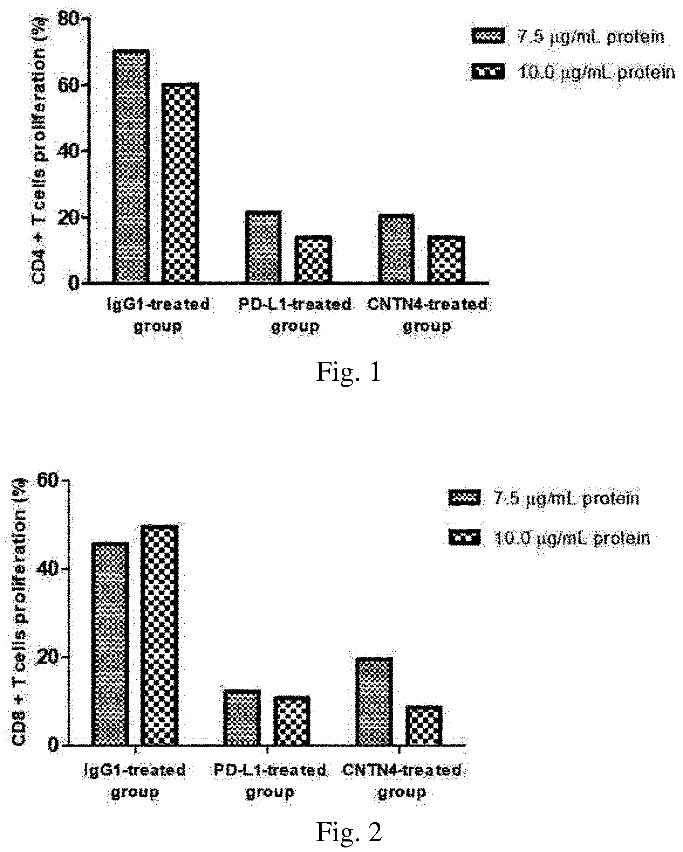

FIG. 1 and FIG. 2 show the percent proliferation (%) of CD4+ T cells and CD8+ T cells, respectively.

The control group treated with PD-L1 inhibited the proliferation of both CD4+ T cells and CD8+ T cells compared to the control group treated with IgG1.

The group treated with CNTN4 remarkably inhibited the proliferation of both CD4+ T cells and CD8+ T cells compared to the control group treated with IgG1. And also, the group treated with CNTN4 inhibited the proliferation of both CD4+ T cells and CD8+ T cells similarly to the control group treated with PD-L1.

It means that if CNTN4 is neutralized by blocking or knockdown, the T cell proliferation inhibition of CNTN4 can be suppressed. Accordingly, the cancer treatment can be effectively achieved.

### Example 2. PBMC cytotoxic function assay

This example is to confirm whether the cytotoxic ability of PBMC against cancer cells is increased when CNTN4 is neutralized using an inhibitor of CNTN4.

### 2.1. Preparation of PBMC

Human blood was placed in a 10 ml tube coated with EDTA (or heparin) and mixed with PBS at a ratio of 1:1. Ficoll-Paque PLUS was placed in a 50 ml tube, and then the blood sample was added. After centrifugation, human PBMCs were collected. The resultant was centrifuged, and the supernatant was removed. Then, RBC lysis (1x) was added, pipetted, and stored on ice for 3 minutes. After that, 50 ml of 10% FBS RPMI1640 was added, and the mixture was centrifuged to remove the supernatant. Then, FACS buffer was added, and the supernatant was removed by centrifugation. Subsequently, 50 ml of MACS buffer (PBS containing 0.5% bovine serum albumin and 2mM EDTA) was added, the number of cells was counted, and the supernatant was completely removed after centrifugation.

96-well plates were coated with 1.0 µg/ml of anti-CD3 antibody (BioLegend, Cat. No. 317325) in PBS at 4°C, and the wells were washed three times with PBS. The PBMC prepared in the above was mixed with 10% FBS RPMI1640, and was added to each well of the 96-well plate at the number of 6×10⁵ cells in an amount of 100 µl. The PBMC was activated by anti-CD3 antibody for 72 hours.

### 2.2. Preparation of cancer cells

Lung cancer cell line A549, colon cancer cell line HCT-116, breast cancer cell line MDA-MB-231, gastric cancer cell line MKN-74, and leukemia cell line U937 were respectively mixed with 1 µl of CFSE (carboxyfluorescein succinimidyl ester), and then stored at 37°C for 3 minutes. Subsequently, FBS was added into tubes containing cancer cells and stored on ice for 10 minutes. Thereafter, the supernatant was removed by centrifugation. The resultant was added with 30 ml of FACS buffer, pipetted, and centrifuged to remove the supernatant. Then, 10% FBS RPMI1640 was added, pipetted, and centrifuged to remove the supernatant. Thereafter, the resultant was mixed with 10 ml of 10% FBS RPMI1640, and the number of cells was counted.

Each PBMC-containing well of the 96-well plate prepared in the Example 2.1 was added with the cancer cells at the number of 3×10⁴ cells in an amount of 100 µl.

### 2.3. Measurement of cytotoxicity of PBMC against cancer cells

The mixtures of PBMCs and cancer cells were prepared in the Example 2.2. These mixtures were incubated for 24 hours with 10 µg/mL of anti-human CNTN4 antibody, or 50 nM of CNTN4 siRNA.

Table 1 below provides the non-treated control group and Groups 1 to 5 using five neutralizing antibodies for blocking CNTN4, and Table 2 below provides the non-treated control group and Groups 6 to 8 using three siRNAs for knockdown of CNTN4.

**Table 1**

| | **human CNTN4 neutralizing antibody** |
|---|---|
| Control group | Not treated |
| Group 1 | anti-human CNTN4 antibody (R&D, MAB2205) |
| Group 2 | anti-human CNTN4 antibody (abeam, ab137107) |
| Group 3 | anti-human CNTN4 antibody (abcam, ab131285) |
| Group 4 | anti-human CNTN4 antibody (LSbio, LS-C119876) |
| Group 5 | anti-human CNTN4 antibody (Abnova, PAB27653) |

**Table 2**

| | **human CNTN4 siRNA** |
|---|---|
| Control group | Not treated |
| Group 6 | Sense (5'-CAGUAUCUUUGCCAGAAGUtt-3') (SEQ ID NO: 1) |
| | Antisense (5'-ACUUCUGGCAAAGAUACUGtt-3') (SEQ ID NO: 2) |
| Group 7 | Sense (5'-GAUAAUGAGUCGGAAGUAAtt-3') (SEQ ID NO: 3) |
| | Antisense (5'-UUACUUCCGACUCAUUAUCtt-3') (SEQ ID NO: 4) |
| Group 8 | Sense (5'-GUGACAAUAGACGAAAUCAtt-3') (SEQ ID NO: 5) |
| | Antisense (5'-UGAUUUCGUCUAUUGUCACtt-3') (SEQ ID NO: 6) |

After twenty-four hours from incubating the mixtures of PBMCs and cancer cells with antibody or siRNA, cells were stained with 7-aminoactinomycin D (7-AAD; BD Pharmingen, San Diego, CA, USA) to detect lysed cells. The cytotoxicity of PBMC against cancer cells was analyzed by determining FL-1 (CFSE) and FL-3 (7-AAD) staining using a FACSDiVa software (BD Biosciences).

### 2.4. Results

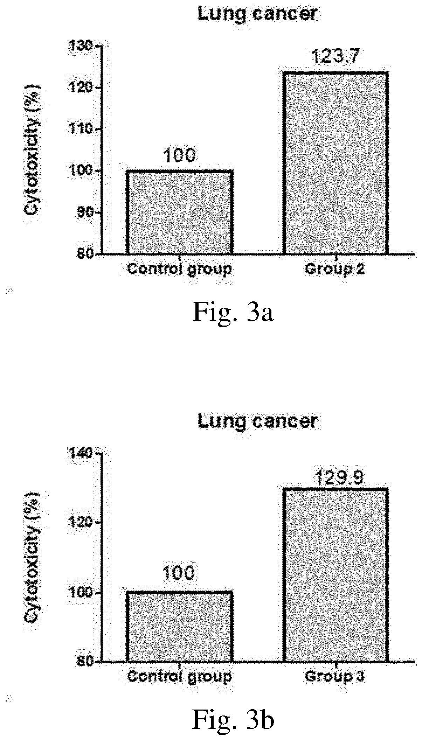

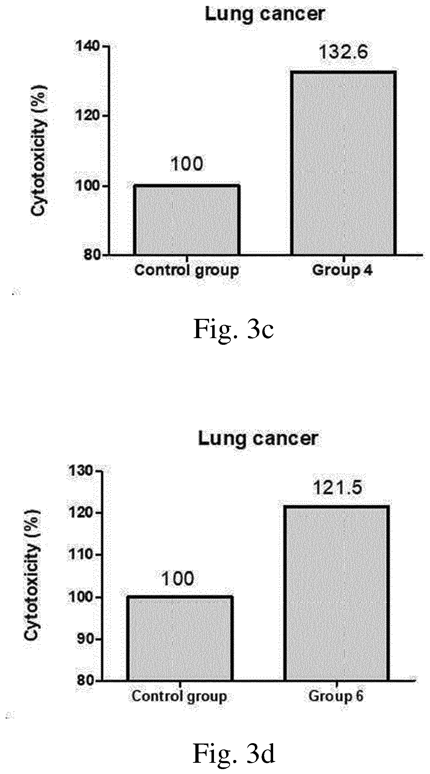

For the lung cancer cell line A549, FIGS. 3a, 3b, 3c and 3d show the results treated with CNTN4 neutralizing antibody or siRNA.

When the lung cancer cell line A549 and PBMC were treated with CNTN4 neutralizing antibody, the cytotoxicity against lung cancer cell was significantly increased compared to the non-treated control group even though there is more or less degree of difference depending on the type of antibody. Further, the cytotoxicity against lung cancer cell was also significantly increased when it was treated with CNTN4 siRNA.

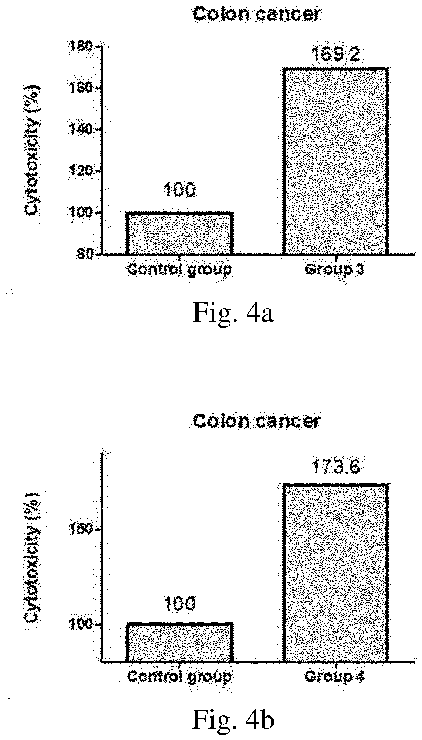

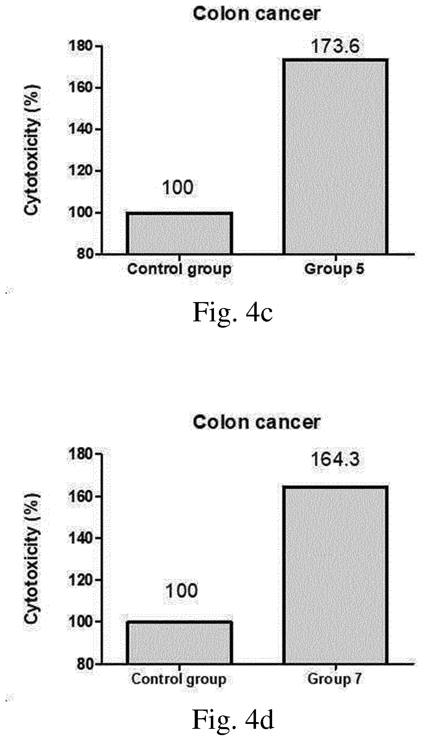

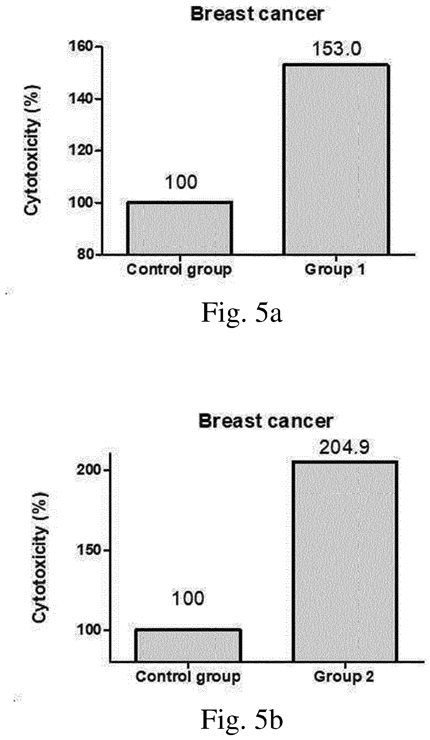

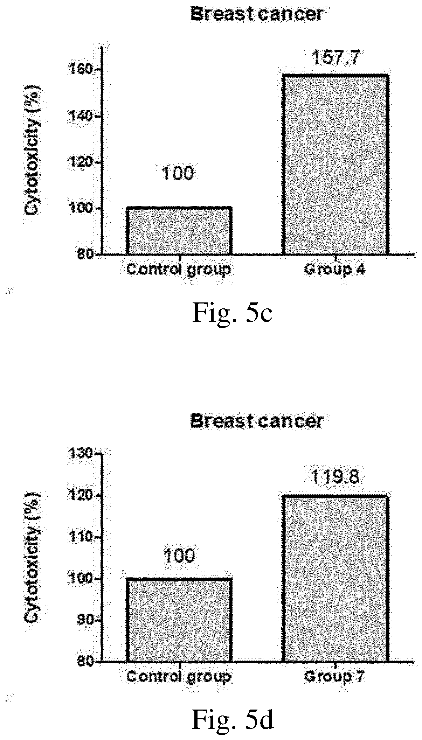

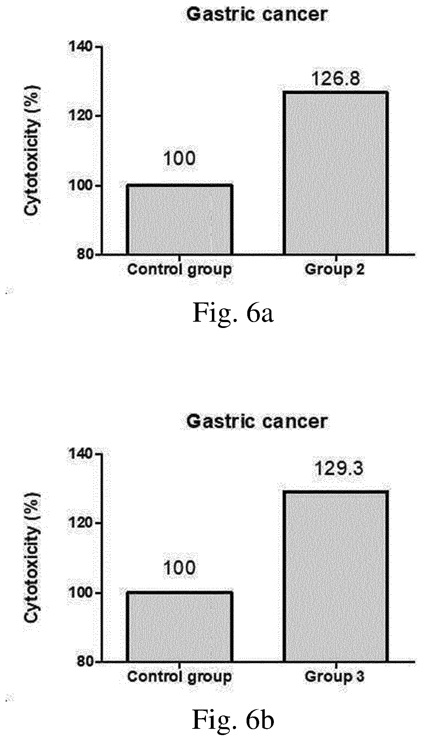

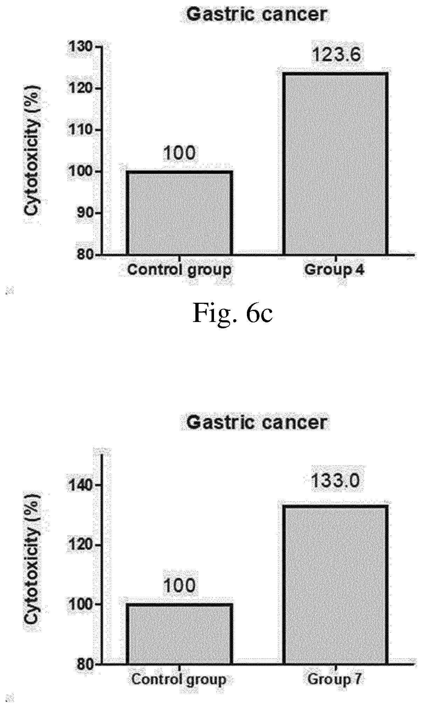

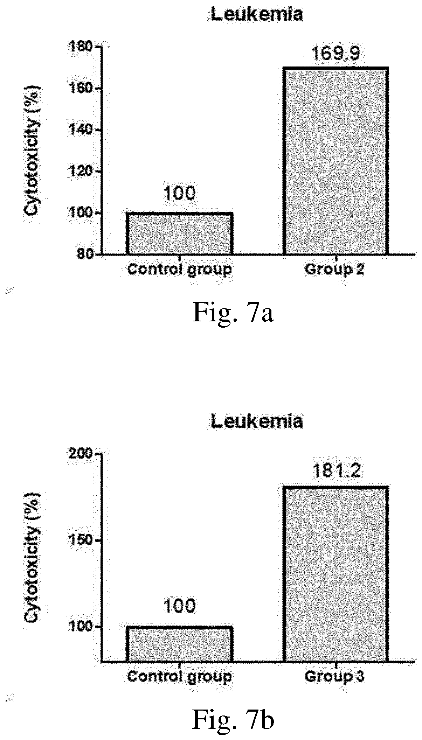

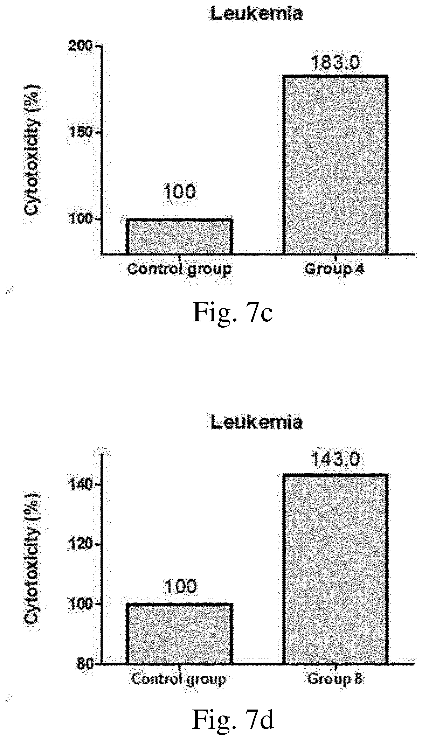

Using CNTN4 neutralizing antibody or siRNA, the results on the colon cancer cell line HCT-116 are shown in FIGS. 4a, 4b, 4c and 4d, the results on the breast cancer cell line MDA-MB-231 are shown in FIGS. 5a, 5b, 5c and 5d, the results on the gastric cancer cell line MKN-74 are shown in FIGS. 6a, 6b, 6c and 6d, and the results on the leukemia cell line U937 are shown in FIGS. 7a, 7b, 7c and 7d.

As shown in FIGS. 3a to 7d, when CNTN4 were neutralized by antibodies or siRNAs, the results of increasing the cytotoxicity of PBMC were also observed in colon cancer, breast cancer, gastric cancer and leukemia.

### Example 3. Tumor mouse model experiment

This example is to confirm whether the growth of tumor in mouse is suppressed when CNTN4 is neutralized using an inhibitor of CNTN4.

### 3.1. Establishment of tumor mouse model

MC-38 cell line derived from C57bL6 colon adenocarcinoma cells was resuspended in 50 µl PBS at the number of 2×10⁵ cells, and was subcutaneously injected into the flanks of 6-week-old female C57bL6 mice.

Table 3 below provides the non-treated control group and Group 9 using a siRNA for knockdown of CNTN4.

**Table 3**

| | **mouse CNTN4 siRNA** |
|---|---|
| Control group | Not treated |
| Group 9 | Sense (5'-GUGUAGACAAACUCUCUGU-3') (SEQ ID NO: 7) |
| | Antisense (5'-ACAGAGAGUUUGUCUACAC-3') (SEQ ID NO: 8) |

In all Groups, the siRNA targeting mouse CNTN4 was injected into the tumor of mice three times at the interval of 5 days from the 11th day after injecting MC-38 cells. Specifically, 10 µg siRNA and 7.5 µl oligofectamine (Invitrogen) in PBS were mixed according to manufacturer's instruction, and then injected into the tumor tissue induced in mice at a dose of 0.5 mg/kg.

### 3.2. Results

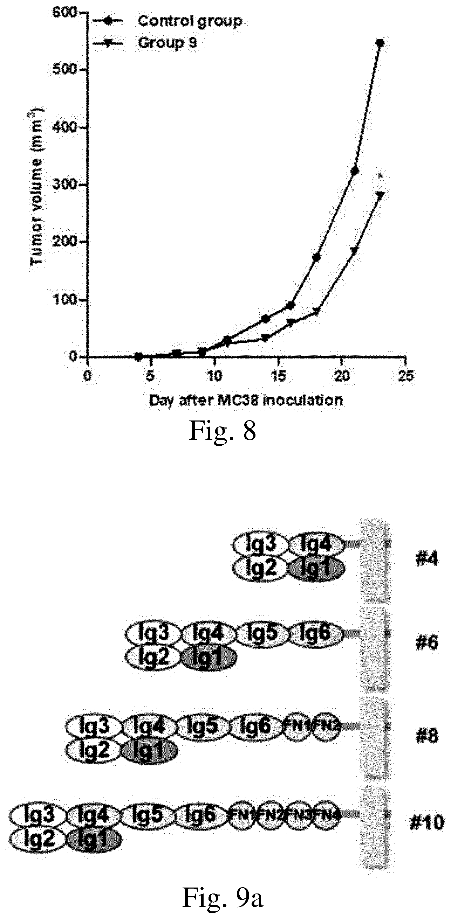

FIG. 8 provides the result on the size of tumor in mice of the non-treated control group and Group 9 wherein CNTN4 was knocked down.

In the non-treated control group, the tumor continued to grow after it occurred. Compared to the non-treated control group, the growth rate of tumor in mouse was remarkably inhibited in Groups wherein CNTN4 was knocked down. It means that when CNTN4 are blocked or knocked down to inhibit its activity or expression, the development of cancer is delayed or stopped and the occurrence of cancer is inhibited. Accordingly, an inhibitor of CNTN4 can be efficiently used to prevent cancer.

### Example 4. Binding site between CNTN4 and APP

This example is to confirm the binding site between CNTN4 and its receptor APP.

### 4.1. Preparation of overexpression cell (Transient transfection)

HEK293FT cells were seeded at the number of 3×10⁶ cells on a culture dish plate. 24 hours later, 200 µL of jetPRIME transfection buffer; 10 µg of CNTN4 expression plasmid or 10 µg of APP expression plasmid; and 20 µL of RIME transfection reagent were mixed and incubated at room temperature (RT) for 10 minutes, and then each mixture was added to the cells to transfect HEK293FT cells with each plasmid.

Herein, four plasmids were prepared and used as CNTN4 expression plasmids. CNTN4 consists of ten domains (i.e., Ig1 to Ig6 and FN1 to FN4). Each plasmid was used for expressing Ig1 to Ig4, Ig1 to Ig6, Ig1 to FN2, and Ig1 to FN4 (Ig; Ig like domain, FN; fibronection). The deletion structures expressed with each plasmid were named #4, #6, #8, and #10 (FIG. 9a).

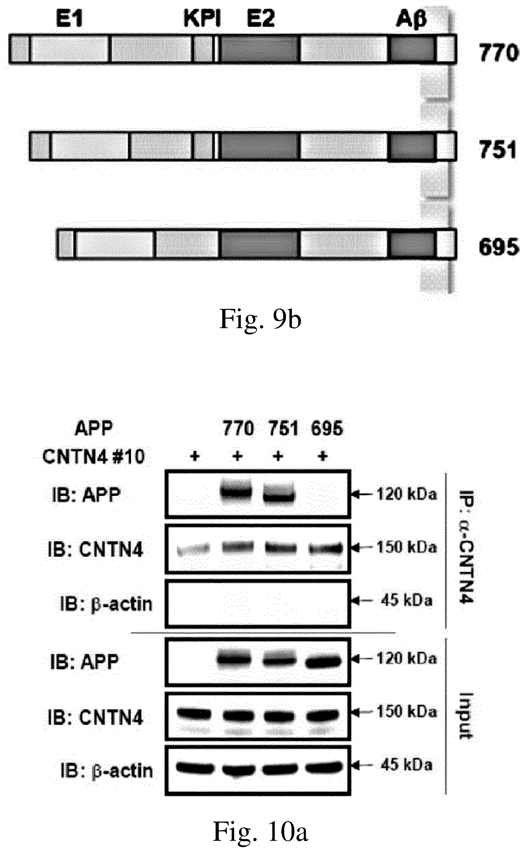

Three plasmids were prepared and used as APP expression plasmids. Each plasmid was used for expressing three isoforms of APP, which were named 770, 751, and 695, respectively. Among them, 770 and 751 isoforms have a KPI (kunitz-type protease inhibitor) domain, and 695 isoform does not have a KPI domain (FIG. 9b).

### 4.2. Immunoprecipitation (IP)

### 4.2.1. Protein to protein interaction

Seven sets of cells transfected in the Example 4.1 were, respectively, centrifuged at 1,200 rpm for 3 min at 4°C. After centrifugation, supernatant was removed and the cell pellet was dissolved with 1 x lysis buffer, and then reacted at 4°C for 10 min. After that, centrifugation was performed again at 13,000 rpm for 10 min at 4°C to obtain cell lysates.

The cell lysate sample was diluted by 1/5 by mixing 6 µL of cell lysate sample and 24 µL of UltraPure distilled water in a new 1.5 mL tube. And also, 2 mg/mL standard BSA was serially diluted by 1/2 with UltraPure distilled water (i.e. 2, 1, 0.5, 0.25, 0.125, 0.0625, 0.031, and 0 mg/mL). 10 µL of each diluted sample and standard BSA was added in duplication to a non-coated 96-well plate. Reagent A and reagent B contained in the BCA assay kit were mixed at a ratio of 50:1, treated 200 µL in each well, and reacted for 30 min at 37°C. The concentration of protein was determined using SpectraMax M2 Microplate Readers.

Then, CNTN4 #10 was reacted with APP 770, 751, and 695 to determine whether a CNTN4-APP protein complex was generated. CNTN4 #4, #6, #8, and #10 were reacted with APP 770 to determine whether a CNTN4-APP protein complex was generated.

100 µg protein of each cell lysate, 500 µL PBS, and 2 mg IP Ab (anti-CNTN4 antibody or anti-APP antibody) were mixed and rotated overnight (O/N) at 4°C.

Then, 30 mL of 50% protein A/G agarose beads were added and rotated at room temperature (RT) for 2 h. After centrifuging at 4,000 rpm for 4 min at 4°C, the supernatant was removed, 1 mL of PBS was added, and the mixture was rotated at RT for 10 min. This process was repeated three times. After centrifuging at 4,000 rpm for 4 min at 4°C, the supernatant was removed, and 20mL of 1x sample buffer prepared by pre-mixing sample buffer and reducing agent was added, and was reacted for 10 min at 95°C heat block. The reactant was placed on ice for 5 min and spun down (IP sample).

Total 20 mg of cell lysates was mixed with sample buffer and reducing agent to make 20 mL, and reacted for 10 min in a 95°C heat block. The reactant was placed on ice for 5 min and spun down (IP sample).

### 4.2.2. SDS-PAGE and western blotting

NuPAGE 4-12% Bis-Tris gel was used in combination with the NuPAGE^{®} electrophoresis system. The gel was fixed to the chamber, and MES running buffer was filled between the gel cassette and electrode assembly. 5mL of marker and sample (20mL per well) were loaded and electrophoresed according to the manufacturer's manual.

The transferred membrane was blocked with 3% BSA blocking buffer at RT for 60 min. After blocking, it was washed three times with PBST, treated with the primary antibody diluted (1:1,000) in 0.3% BSA blocking buffer, and placed in a refrigerator overnight at 4°C. After washing three times with PBST, and treating with the secondary antibody diluted (1:5,000) in 0.3% BSA blocking buffer, it was reacted at RT for 60 min. Then, it was washed three times with PBST.

500 µL of Luminol/Enhancer solution and 500 µL of peroxide solution of SuperSignal^{™} West Pico PLUS Chemiluminescent Substrate were mixed in a 1.5 mL tube, sprayed on the membrane, and photographed using LOURMAT CHEMI-DOC.

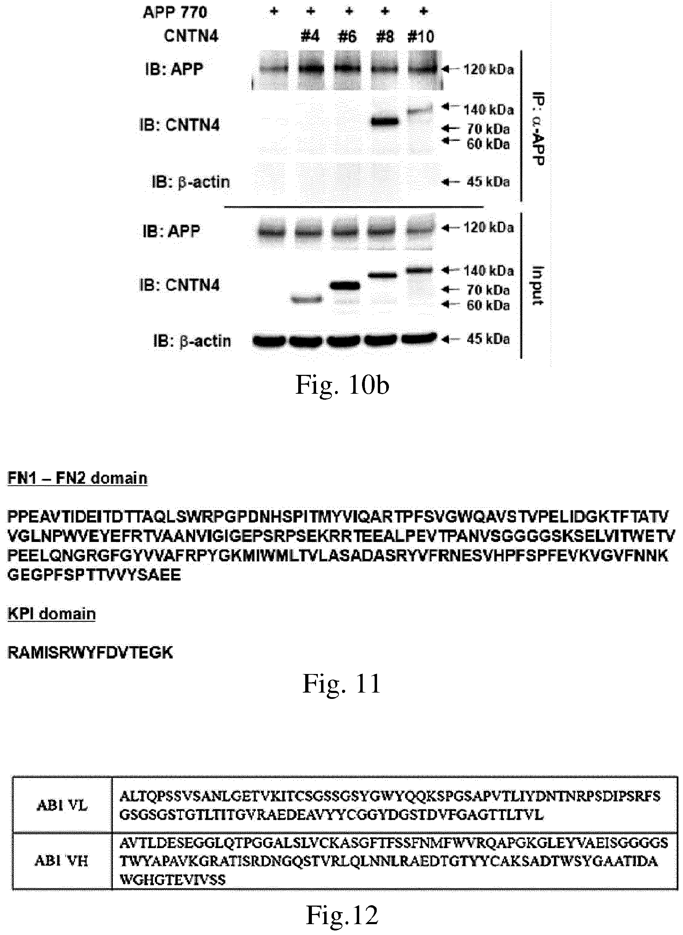

The results are shown in FIG. 10.

CNTN4 #10 (full length) binds to APP (770 & 751) which has the KPI (kunitz-type protease inhibitor) domain (FIG. 10a). APP 770 (full length) binds to CNTN4 (#8 & #10) which has the FN1 ~ FN2 domain (domain 7 ~ domain 8) (FIG. 10b). It means that the APP site to which CNTN4 binds is the KPI domain, and the corresponding CNTN4 binding site is the FN1 to FN2 domain (domain 7 to domain 8). The sequence of each domain is shown in Table 4 and FIG. 11.

**Table 4**

| | | |
|---|---|---|
| SEQ ID NO: 9 | FN1 to FN2 domain | PPEAVTIDEITDTTAQLSWRPGPDNHSPITMYVIQA RTPFSVGWQAVSTVPELIDGKTFTATVVGLNPWV EYEFRTVAANVIGIGEPSRPSEKRRTEEALPEVTPA NVSGGGGSKSELVITWETVPEELQNGRGFGYVVA FRPYGKMIWMLTVLASADASRYVFRNESVHPFSP FEVKVGVFNNKGEGPFSPTTVVYSAEE |
| SEQ ID NO: 10 | KPI domain | RAMISRWYFDVTEGK |

### Example 5. Antibody binding site of CNTN4 using anti-CNTN4 antibody (AB1)

This example is to confirm the antibody binding site of CNTN4 by confirming the binding between the anti-CNTN4 antibody (AB1) and the CNTN4 domain.

### 5.1. Preparation of anti-CNTN4 antibody AB 1

### 5.1.1. scFv antibody library preparation and selection

Antibodies were produced by inoculating 15 chickens with mouse CNTN4 and/or human CNTN4. Four animals who were confirmed to produce antibodies through ELISA were selected. After sacrificing them, blood, spleen, bone marrow, and bursa fabricius were obtained, total RNA was extracted, and cDNA was synthesized. scFv fragments were obtained by linking the variable regions of the light and heavy chains of the antibody with a linker through PCR. Then, phage display was performed. Through phase ELISA screening, a positive clone binding to CNTN4 was selected, and the corresponding scFv sequence was selected (scFv AB 1). This was prepared and expressed as an scFv labeled with constant human kappa (hCk), a fusion protein, and its binding ability to CNTN4 was confirmed. Table 5 and FIG. 12 show the amino acid sequences of the light chain and heavy chain variable regions of the selected scFv AB 1.

**Table 5**

| | | |
|---|---|---|
| SEQ ID NO: 11 | Variable region of light chain (VL) | ALTQPSSVSANLGETVKITCSGSSGSYGWYQQ KSPGSAPVTLIYDNTNRPSDIPSRFSGSGSGSTG TLTITGVRAEDEAVYYCGGYDGSTDVFGAGT TLTVL |
| SEQ ID NO: 12 | Variable region of heavy chain (VH) | AVTLDESEGGLQTPGGALSLVCKASGFTFSSF NMFWVRQAPGKGLEYVAEISGGGGSTWYAP AVKGRATISRDNGQSTVRLQLNNLRAEDTGT YYCAKSADTWSYGAATIDAWGHGTEVIVSS |

### 5.1.2. Preparation of anti-CNTN4 chicken-human chimeric IgG4 antibody AB1

The light and heavy chain regions of the scFv selected in the Example 5.1.1 were cloned into a bicistronic expression vector. DNA was transiently transfected into Expi293F cells and cultured to a viability of 50% to express IgG. The culture medium was bound to Kappa select resin (kappa region capture) to elute the IgG and subject to primary purification. Afterwards, it was bound to Mabselect (Fc region capture) to remove light chain impurities, and the IgG was eluted and subjected to secondary purification. An anti-CNTN4 129 chimeric IgG4 antibody (hereinafter referred to as "AB1") with a purity of 72.4% on SEC-HPLC was produced.

### 5.2. Antibody binding site of CNTN4

### 5.2.1. Preparation of human CNTN4 domain sample

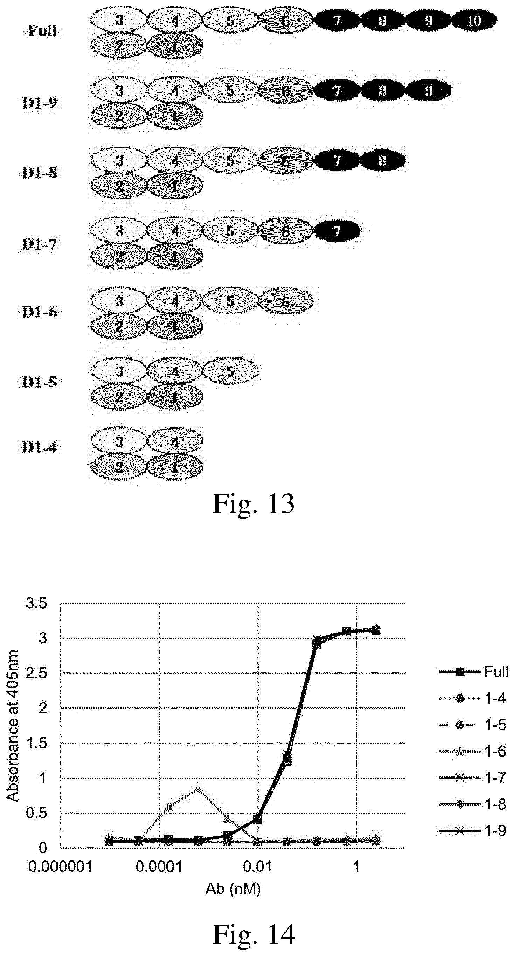

Human CNTN4 domain sample was prepared according to FIG. 13. CNTN4 consists of ten domains (i.e., Ig1 to Ig6 and FN1 to FN4; indicated as Domains 1 to 10 in FIG. 13, respectively), and Full indicates a sample containing all of the ten domains. D1-9 is a sample in which the domain 10 was deleted from Full, and D1-8 is a sample in which the domain 9 was deleted in D1-9. Domain samples D1-4 to D1-7 were prepared in the same manner.

The full domain concentration was set as 4 µg/mL. The concentration of each domain was determined according to the molecular weight ratio to apply the same molar concentration as the full domain (Table 6).

**Table 6**

| **Domain Number** | Full | 1-4 | 1-5 | 1-6 | 1-7 | 1-8 | 1-9 |
|---|---|---|---|---|---|---|---|
| **Concentration (µg/mL)** | 4 | 1.6 | 2.0 | 2.3 | 2.8 | 3.2 | 3.6 |
| **Molecular Weight (kDa)** | 111.08 | 44.43 | 54.48 | 64.83 | 76.83 | 87.93 | 99.03 |

### 5.2.2. Binding of antibody AB 1 and CNTN4 domain sample

50 µL/well of the CNTN4 domain prepared in the Example 5.2.1 was dispensed to a 96-well plate. After sealing with sealing tape, it was incubated overnight at 4°C. The next day, the sealing tape was removed, and the buffer was removed. 150 µL of blocking buffer was dispensed into each well, sealed, and incubated for 1 hour at 37°C.

Afterwards, the blocking buffer was removed, and 50 µL of each primary antibody (antibody AB 1) diluted with blocking buffer according to Table 13 was dispensed to each well. It was sealed with sealing tape and incubated for 2 hours at 37°C. After removing the sealing tape, washing was performed three times.

**Table 7**

| **Tube Number** | 1 | 2 | 3 | 4 | 5 | 6 | 7 | 8 | 9 |
|---|---|---|---|---|---|---|---|---|---|
| **Cone. (nM)** | 2.5 | 0.625 | 0.156 | 0.0391 | 0.0097 7 | 0.0024 4 | 0.0006 1 | 0.0001 53 | 0.0000 4 |

50 µL of diluted secondary antibody (anti-human IgG Fc-HRP) was dispensed to each well. It was sealed with sealing tape and incubated for 1 hour at 37°C. After removing the sealing tape, washing was performed three times. 50 µL of ABTs were dispensed to each well, incubated at room temperature for 30 minutes, and absorbance at 405 nm was measured using a microplate reader. Table 8 and FIG. 14 show the results.

**Table 8**

| **Domain** | Full | 1-4 | 1-5 | 1-6 | 1-7 | 1-8 | 1-9 |
|---|---|---|---|---|---|---|---|
| **Binding** | + | - | - | - | - | + | + |

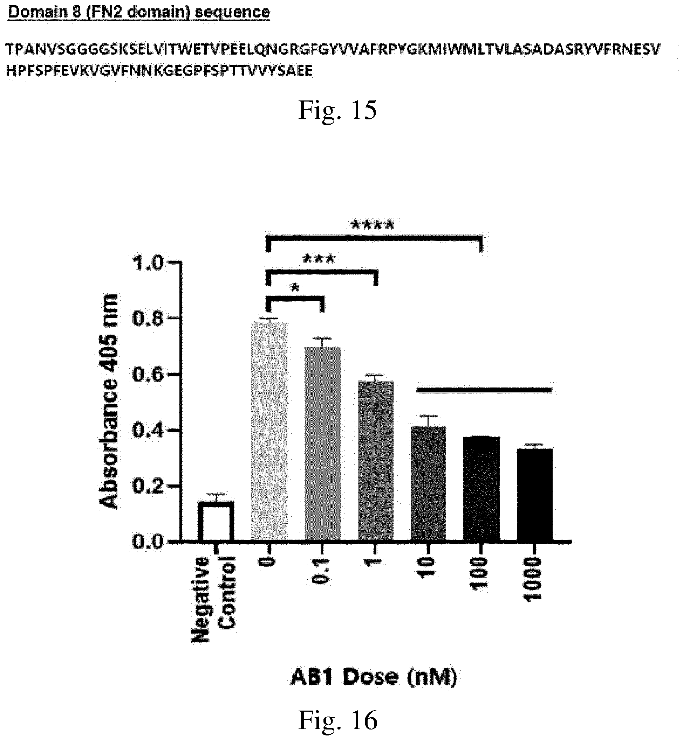

Anti-CNTN4 antibody AB1 bound only to D1-8, D1-9, and Full among the seven Human CNTN4 domain samples (the three graphs overlap each other in FIG. 14), but did not bind to D1-4 to D1-7 (the four graphs overlap each other, except for D1-6 at antibody concentrations of approximately 0.0001 to 0.01 nM). Considering that D1-7 and D1-8 differ in the presence or absence of Domain 8, it can be confirmed that Domain 8 (i.e., FN2) is an important site for the binding of CNTN4 and anti-CNTN4 antibodies. Table 9 and FIG. 15 show the amino acid sequence of Domain 8 of CNTN4.

**Table 9**

| | | |
|---|---|---|
| SEQ ID NO: 13 | Domain 8 (FN2 Domain) | TPANVSGGGGSKSELVITWETVPEELQNGRGFGYVVA |
| | | FRPYGKMIWMLTVLASADASRYVFRNESVHPFSPFEV KVGVFNNKGEGPFSPTTVVYSAEE |

### Example 6. Competitive binding between APP and antibody AB1 for CNTN4

This example is to confirm whether the binding between APP and CNTN4 is blocked by anti-CNTN4 antibody AB1.

First, 4 mg/mL of CNTN4 was coated on the plate. APP (his tagged, 0.64mg/mL) was diluted to 40mg/mL using ELISA buffer. And, anti-CNTN4 antibody AB1 (4.98mg/mL; 35mM) was serially diluted 1/10 to 1 to 0mM using ELISA buffer. Diluted APP and AB1 antibodies were each treated at 25 mL/well to a 96-well half plate coated with CNTN4, reacted for 4 hours at room temperature (proceeded in duplicate), and washed. Anti-his tag was diluted to 1:2,000 in ELISA buffer, treated at 50 mL/well, incubated for 1 hour at room temperature, and washed. Subsequently, 50 mL/well of ABTS was treated, and the absorbance was measured at 405 nm using a plate reader.

FIG. 16 shows the results.

It was confirmed that the absorbance decreased as the concentration of AB 1 increased when 40 mg/mL APP and antibody AB 1 at various concentrations were treated in the plate coated with 4 mg/mL CNTN4. This indicates that antibody AB1 against CNTN4 competitively binds to APP and blocks the binding of APP to CNTN4.

## Claims

1. A pharmaceutical composition for treating or preventing cancer, comprising a CNTN4 inhibitor,
wherein the CNTN4 inhibitor is an antisense nucleic acid, a siRNA, a shRNA, a miRNA or a ribozyme that binds in a complementary manner to a DNA or a mRNA of CNTN4 gene; or a compound, a peptide, a peptide mimetic, a fusion protein, an antibody or antigen-binding fragment thereof, an Antibody Drug Conjugate (ADC) or an aptamer that binds specifically to CNTN4 protein.

2. The pharmaceutical composition for treating or preventing cancer according to claim 1, wherein the cancer is stomach cancer, lung cancer, liver cancer, colorectal cancer, colon cancer, small intestinal cancer, pancreatic cancer, brain cancer, bone cancer, melanoma, breast cancer, sclerosing adenosis, uterine cancer, cervical cancer, head and neck cancer, esophageal cancer, thyroid cancer, parathyroid cancer, renal cancer, sarcoma, prostate cancer, urethral cancer, bladder cancer, blood cancer, lymphoma, or fibroadenoma.

3. The pharmaceutical composition for treating or preventing cancer according to claim 1, wherein the CNTN4 inhibitor suppresses a function of cancer cells evading T cells.

4. A pharmaceutical composition for immune-enhancing in a subject to increase a level of T cell-mediated immune response, comprising a CNTN4 inhibitor,
wherein the CNTN4 inhibitor is an antisense nucleic acid, a siRNA, a shRNA, a miRNA or a ribozyme that binds in a complementary manner to a DNA or a mRNA of CNTN4 gene; or a compound, a peptide, a peptide mimetic, a fusion protein, an antibody or antigen-binding fragment thereof, an Antibody Drug Conjugate (ADC) or an aptamer that binds specifically to CNTN4 protein.

5. The pharmaceutical composition for immune-enhancing according to claim 4,
wherein the subject is in need of prevention, treatment or improvement of diseases related to immunodeficiency, decreased immunity or immune system damage.

6. A method of screening an anti-cancer agent comprising:
(a) contacting a cancer cell with a candidate anti-cancer agent; and
(b) measuring expression or activity of CNTN4 in the cancer cell.

7. The method of screening an anti-cancer agent according to claim 6,
wherein the step (b) is conducted by determining an expression level of mRNA or protein of CNTN4 or a suppression level of T cell activity by CNTN4.

8. The method of screening an anti-cancer agent according to claim 6, further comprising: determining the candidate anti-cancer agent to be the anti-cancer agent if compared to a group not treated with the candidate anti-cancer agent, a group treated with the candidate anti-cancer agent shows a significantly lower level of expression of mRNA or protein of CNTN4, or a significantly lower level of suppression of T cell activity by CNTN4.

9. A pharmaceutical composition for treating or preventing cancer, comprising an anti-CNTN4 antibody or antigen-binding fragment thereof that binds to the Fibronectin 2 domain of CNTN4.

10. The pharmaceutical composition for treating or preventing cancer according to claim 9, wherein the Fibronectin 2 domain comprises an amino acid sequence of SEQ ID NO: 13.

11. The pharmaceutical composition for treating or preventing cancer according to claim 9, wherein the anti-CNTN4 antibody or antigen-binding fragment thereof blocks or inhibits binding of CNTN4 to amyloid precursor protein (APP).

12. A use of a CNTN4 inhibitor for treating or preventing cancer,
wherein the CNTN4 inhibitor is an antisense nucleic acid, a siRNA, a shRNA, a miRNA or a ribozyme that binds in a complementary manner to a DNA or a mRNA of CNTN4 gene; or a compound, a peptide, a peptide mimetic, a fusion protein, an antibody or antigen-binding fragment thereof, an Antibody Drug Conjugate (ADC) or an aptamer that binds specifically to CNTN4 protein.

13. A method of treating or preventing cancer, comprising administering a CNTN4 inhibitor to a subject in need thereof,
wherein the CNTN4 inhibitor is an antisense nucleic acid, a siRNA, a shRNA, a miRNA or a ribozyme that binds in a complementary manner to a DNA or a mRNA of CNTN4 gene; or a compound, a peptide, a peptide mimetic, a fusion protein, an antibody or antigen-binding fragments thereof, an Antibody Drug Conjugate (ADC) or an aptamer that binds specifically to CNTN4 protein.
